# EUROPEAN PATENT APPLICATION

(11) **EP 3 219 797 A1**
(43) Date of publication of application: **20.09.2017**
(21) Application number: 15858911.9
(22) Date of filing: 10.11.2015
(51) Int. Cl.: C12N 15/00, C07K 14/195, C12N 1/15, C12N 1/22, C12N 15/09, C12P 19/14

(54) **CELLULASE ACTIVATOR AND METHOD FOR SACCHARIFYING LIGNOCELLULOSIC BIOMASS BY USING SAME**

(30) Priority: 12.11.2014 JP 2014230027
(71) Applicant: Riken, Wakou-shi, Saitama 351-0198 (JP)
(72) Inventor: MORIYA, Shigeharu, Wako-shi Saitama 351-0198 (JP); OTAGIRI, Masato, Wako-shi Saitama 351-0198 (JP); YUKI, Masahiro, Wako-shi Saitama 351-0198 (JP); OHKUMA, Moriya, Wako-shi Saitama 351-0198 (JP); KISHIKAWA, Shotaro, Wako-shi Saitama 351-0198 (JP)
(74) Representative: Denison, Christopher Marcus
(86) International application number: PCT/JP2015/081530
(87) International publication number: WO 2016/076282

(57) **Abstract**

The present invention relates to developing and providing a technique for saccharifying a plant biomass, particularly a lignocellulosic biomass efficiently in fewer steps and at low cost while suppressing environmental load, in order to efficiently and effectively use the energy of the plant biomass.

Provided are a cellulase activator containing a lignocellulose degradation cofactor consisting of an amino acid sequence represented by SEQ ID NO: 1 as an active ingredient and a method for saccharifying a lignocellulosic biomass using the cellulase activator.

## Description

### Technical Field

The present invention relates to a cellulase activator containing a novel lignocellulose degradation cofactor as an active ingredient and a method for saccharifying a lignocellulosic biomass using the cellulase activator.

### Background Art

Recently, a move to produce a bio fuel such as bioethanol, biodiesel and biogas by using a plant biomass abundantly present on the earth as alternative energy resource to a fossil fuel, has been accelerated in all over the world. The plant biomass is a recyclable resource and has an advantage of being free of a depletion problem, unlike a fossil fuel. In addition, the energy of the plant biomass is derived from atmospheric carbon dioxide, which is generated by combustion and fixed by photosynthesis. Due to this, the energy of the plant biomass is carbon neutral circulation energy and can contribute to prevention of global warming.

However, bio fuel production using a plant biomass has several problems to be solved. For the raw material for bioethanol and biodiesel, at present, corn, soybean or residue of juice of sugar cane (bagasse) is mainly used. However, since corn and soybean are a food plant biomass, and thus, the raw material of bio fuel competes with food and feed. In the result, a price increase at a world-wide level is a matter of concern. As a result of influence by market prices, it is difficult to stably keep necessary amounts of them. As to sugar cane, the temperate zone and cool-temperature zone cannot be used as a cultivation area. In addition to these problem, an environmental destruction such as razing the forest along with an increase of cultivated land has been a big issue.

In the circumstances, instead of the food plant biomass, a non-food plant biomass such as a lignocellulosic biomass has attracted attention as a new plant biomass now. The lignocellulosic biomass, which constitutes woody part of plants, is said to be a biomass most abundantly present on earth. Furthermore, because of the non-food plant biomass, competition with food will not occur. Moreover, raw materials thereof can be stably and inexpensively obtained. Because of this advantage, its potential value of use is extremely high.

However, production of bio fuel using a lignocellulosic biomass also has problems in view of conversion yield and manufacturing cost. Generally, in order to use a lignocellulosic biomass as a bio fuel, a saccharification reaction is essential to convert lignocellulose into a saccharide such as a monosaccharide or disaccharide; however, since lignocellulose contains a chemically very stable cellulose, hemicellulose and lignin as main components, saccharification treatment is not easy. Conventionally, for industrially saccharifying a lignocellulosic biomass, an acid treatment method is employed (Non Patent Literature 1). The acid treatment method involves in steaming a lignocellulosic biomass together with an acid such as dilute sulfuric acid and hydrochloric acid, followed by distilling the resultant biomass. However, this method puts a large environmental load and requires a lot of energy. Thus, this method has a problem of high cost.

### Citation List

### Non Patent Literature

Non Patent Literature 1: 1612 chemical products published by The Chemical Daily Co., Ltd., p 871, (2012)

### Summary of Invention

### Technical Problem

The problem to be solved by the present invention is to develop and provide a technique for saccharifying a plant biomass, particularly a lignocellulosic biomass efficiently in fewer steps and at low cost while reducing environmental load, in order to efficiently and effectively use the energy of the plant biomass.

### Solution to Problem

To solve the above problem, the present inventors employed not a conventional acid treatment method but a biological degradation method using an enzyme as a saccharification method for a lignocellulosic biomass.

Cellulose is a polysaccharide present in lignocellulose in an amount of about 50% and hydrolyzed by cellulase into e.g., β-D-glucose. However, if a reaction is carried out just by adding cellulase simply to a lignocellulosic biomass, the degradation efficiency thereof is very low. This is considered because lignin, which is tightly bound to cellulose in lignocellulose, inhibits binding of cellulase and cellulose and degradation of cellulose. Accordingly, in order to efficiently degrade a lignocellulosic biomass by use of cellulase, it is necessary to remove lignin from the lignocellulosic biomass in advance. Lignin can be removed by an acid hydrolysis treatment or a solubilization treatment with an organic solvent; however, problems to be solved by the present invention, i.e., reducing the number of production steps and environmental load, cannot be solved by the method.

In the meantime, insects of the *Isoptera* or insects of the *Cryptocercus* have acquired an advanced lignocellulosic degradation ability to separate lignin from lignocellulose and efficiently degrade cellulose and hemicellulose for using them. The ability is principally due to an enzyme and a cofactor thereof, which are produced by a flagellate (symbiotic flagellate in the digestive tract) symbiotically living in the hindgut of e.g., an insect of the *Isoptera,* ever more than the enzyme produced by these insects themselves. Accordingly, if an enzyme and a cofactor involved in a lignocellulose degradation mechanism, which are obtained by isolating the flagellate from the intestine of *Isoptera* or *Cryptocercus* and culturing it, are used, a lignocellulosic biomass can be efficiently saccharified. However, it is difficult to culture the flagellate symbiotically living in the intestine, in the same manner as in general environmental microorganisms. Also specific degradation mechanism of lignocellulose in a flagellate has been almost unknown.

In the circumstances, the present inventors carried out metatranscriptomic analysis directed to all symbiotic flagellates living in the digestive tract of *Neotermes koshunensis* and *Coptotermes formosanus* instead of isolating and culturing the flagellates to search a highly active cellulase and a non-cellulase biomass degradation cofactor. As a result, a novel lignocellulose degradation cofactor that has not yet been reported was obtained from these symbiotic flagellate groups living in the digestive tracts of *Isoptera.* They further found that if the cofactor is added in combination with cellulase to a lignocellulose biomass, the cellulase activity can be enhanced and saccharification of the lignocellulosic biomass can be accelerated. The invention of the present application was made as the results of the aforementioned studies and is more specifically as follows.

(1) A polypeptide consisting of any one of the following amino acid sequences (a) to (c) or an active fragment thereof:
   (a) an amino acid sequence represented by SEQ ID NO: 1 or 9,
   (b) an amino acid sequence obtained by deleting, substituting or adding one or more amino acids in the amino acid sequence represented by SEQ ID NO: 1 or 9, and
   (c) an amino acid sequence having an amino acid identity of 90% or more with the amino acid sequence represented by SEQ ID NO: 1 or 9.
(2) A polynucleotide encoding the polypeptide or an active fragment thereof according to (1).
(3) The polynucleotide according to (2), consisting of any one of the following nucleotide sequences (d) to (g):
   (d) a nucleotide sequence represented by SEQ ID NO: 2 or 10,
   (e) a nucleotide sequence obtained by deleting, substituting or adding one or more nucleotides in the nucleotide sequence represented by SEQ ID NO: 2 or 10,
   (f) a nucleotide sequence having a nucleotide identity of 90% or more with the nucleotide sequence represented by SEQ ID NO: 2 or 10, and
   (g) a nucleotide sequence hybridizing with a part of the nucleotide sequence complementary with the nucleotide sequence represented by SEQ ID NO: 2 or 10, under stringent conditions.
(4) An expression vector comprising the polynucleotide according to (2) or (3) expressibly.
(5) A transformant obtained by introducing the expression vector according to (4) into a host, or a progeny thereof.
(6) The transformant or a progeny thereof according to (5), wherein the host is a *Trichoderma* filamentous fungus.
(7) A cellulase activator comprising the polypeptide according to (1) as an active ingredient.
(8) A method for saccharifying a lignocellulosic biomass, comprising a cellulase mixing step of mixing cellulase with the lignocellulosic biomass; a cellulase activator mixing step of mixing the cellulase activator according to (7) with the lignocellulosic biomass; and a reaction step of bringing the lignocellulosic biomass and the cellulase into contact with each other in the presence of the cellulase activator to hydrolyze lignocellulose.
(9) A method for producing a reducing sugar from a lignocellulosic biomass using the method according to (8).

The specification incorporates the contents disclosed in JP Application No. 2014-230027, based on which this application claims priority.

### Advantageous Effects of Invention

The cellulase activator according to the present invention allows a lignocellulosic biomass to be efficiently saccharified in fewer steps at low cost by applying the cellulase activator together with cellulase to the biomass.

### Brief Description of Drawings

[Figure 1] The figure is a diagram showing how to construct an expression vector of the present invention, p2636A49-101.
[Figure 2] The figure is a graph showing the results of a saccharification reaction of napier grass using 2636A49 enzyme solution. "A" represents commercially available Cellic (registered trade mark) CTec2 (Novozymes); "B" represents vector pBI101 alone; and "C" represents 2636A49 enzyme solution.
[Figure 3] The figure is a graph showing the results of a saccharification reaction of rice straw using 1028A66 enzyme solution. "A" represents an enzyme solution prepared from *Trichoderma* ATCC66589 strain alone; "B" represents 1028A66 enzyme solution. Reference numbers #1 to #3 of "B" represent transformants of *Trichoderma* ATCC66589 strain independently screened by p1028A66.

### Description of Embodiments

### 1. Lignocellulose degradation cofactor or active fragment thereof

### 1-1. Outline and definition

A first aspect of the present invention is a lignocellulose degradation cofactor or an active fragment thereof (hereinafter sometimes referred to as the "lignocellulose degradation cofactor or the like"). The cofactor or an active fragment thereof of the present invention itself may contribute to an efficient and low-cost saccharification reaction of a lignocellulosic biomass as a formulation of enhancing and accelerating cellulose saccharification ability of cellulase and as an active ingredient of the cellulase activator of the present invention (described later).

"Lignocellulose" is a constituent of the cell wall of a plant and a plant fiber and mainly comprises cellulose, hemicellulose and lignin.

In the specification, "a lignocellulosic biomass" refers to an organic resource composed of lignocellulose. For example, woody biomass, woody waste, herbaceous biomass and herbaceous waste correspond to it. Examples of the woody biomass include remaining materials of forest land of needle-leaved trees or broad-leaved trees, thinned wood, unused trees, short-cycle cultivated wood, and pruned wood of trees in e.g., streets and parks. Examples of the woody waste include building waste materials, lumber remaining materials (including bark, saw dust, planer debris and short timber), and paper resources (for example, newspaper, magazine and waste OA paper, cardboard). Examples of the herbaceous biomass include agricultural plant waste (for example, such as stem, leaf and grain crust, more specifically, rice straw, wheat straw, rice husk, wheat husk, cotton seed husk, bran; stem, leave and cob of corn; and stem and leave of sorghum). Examples of the herbaceous waste include juice residue of plant bodies (including beer cake, bagasse, beet pulp, okara, cotton seed meal and oil cake).

"Cellulose" is a polysaccharide represented by a molecular formula, (C₆H₁₀O₅)ₙ and a compound produced by linear polymerization of e.g., β-D-glucose via a glycosidic bond. The abundance ratio of cellulose in lignocellulose is usually about 50%, as described above.

"Hemicellulose" is a collective term of insoluble polysaccharides except cellulose. For example, xylan, mannan, glucuronoxylan and glucomannan correspond to this. The abundance ratio of hemicellulose in lignocellulose, which varies depending upon the plant from which the lignocellulose is derived, is usually around 30%.

"Lignin" is a phenolic polymer. In lignocellulose, lignin binds to cellulose and hemicellulose and solidifies, in other words, serves like an adhesive. The abundance ratio of lignin in lignocellulose, which varies depending upon the plant and site from which the lignocellulose is derived, similarly to hemicellulose, is usually 20 to 30%. The abundance ratio thereof in sites such as leaves in which lignification is not advanced is usually further lower.

In the specification, "cellulase" is a collective term of enzymes hydrolyzing a β-1,4-glucan bond or a β-D-glucosidic bond of cellulose to produce cello-oligosaccharide, cellobiose and β-D-glucose. Cellulases are classified into three types: endoglucanase, exoglucanase (cellobiohydrolase) and β-glucosidase (β-D-glucoside glucohydrolase), depending upon the mechanism of action. In the specification, unless otherwise specified, any type of cellulase may be used and the type of cellulase is not particularly limited.

### 1-2. Constitution

In the specification, "lignocellulose degradation cofactor" is a non-enzymatic protein involved in degradation of lignocellulose and refers to a factor having an auxiliary function of being capable of enhancing cellulase activity in lignocellulose degradation if it is used in combination with cellulase, although the cofactor alone rarely or never has lignocellulose degradation activity.

In the specification, the lignocellulose degradation cofactor is the polypeptide consisting of the amino acid sequence represented by SEQ ID NO: 1 or 9. The polypeptide is a novel protein, which was obtained by metatranscriptomic analysis from the symbiotic flagellate group living in the digestive tracts derived from *Neotermes koshunensis* and *Coptotermes formosanus.* The polypeptide consisting of the amino acid sequence represented by SEQ ID NO: 1 has cellulose and carbohydrate binding modules. Although the mechanism of action thereof is unknown, it was found herein that the polypeptide can enhance cellulase activity if it is used in combination with cellulase to accelerate saccharification of a lignocellulosic biomass. The polypeptide consisting of the amino acid sequence represented by SEQ ID NO: 9 is predicted as xylanase/chitin deacetylase based on the homology with the amino acid sequence. Similar to the above polypeptide, it was found herein that the polypeptide can enhance cellulase activity if it is used in combination with cellulase to accelerate saccharification of a lignocellulosic biomass.

In the specification, as the lignocellulose degradation cofactor, other than the above polypeptides, a polypeptide consisting of an amino acid sequence obtained by deleting, substituting or adding one or more amino acids in the amino acid sequence represented by SEQ ID NO: 1 or 9; or a polypeptide consisting of an amino acid sequence having an amino acid identity of 90% or more, preferably 95% or more, more preferably 97% or more, 98% or more or 99% or more with the amino acid sequence represented by SEQ ID NO: 1 or 9 and having an activity as a cofactor for enhancing cellulase activity, may be mentioned. In the specification, "a plurality of" refers to the number of, for example, 2 to 20, 2 to 15, 2 to 10, 2 to 7, 2 to 5, 2 to 4 or 2 to 3. The "amino acid identity" refers to the ratio (%) of the number of same amino acid residues relative to the total number of the amino acid residues of the protein represented by SEQ ID NO: 1 or 9, when two amino acid sequences are aligned so as to obtain a maximum degree of similarity of amino acids of them, if necessary, by introducing a gap(s) in either one of the amino acid sequences. The amino acid identity can be computationally obtained by using a protein search system such as BLAST and FASTA (Karlin, S. et al., 1993, Proc. Natl. Acad. Sci. USA, 90: 5873-5877; Altschul, S. F. et al., 1990, J. Mol. Biol., 215: 403-410; Pearson, W. R. et al., 1988, Proc. Natl. Acad. Sci. USA, 85: 2444-2448).

In the specification, "active fragment thereof" is an active fragment of the lignocellulose degradation cofactor, more specifically, refers to a polypeptide fragment containing a part of the amino acid sequence of the cofactor and having an activity as the cofactor. The length of amino acid sequence of the active polypeptide fragment is not particularly limited, as long as the fragment has the activity of the lignocellulose degradation cofactor of the present invention.

In the lignocellulose degradation cofactor and an active fragment thereof, one or more of amino acids may be modified with e.g., methylation. Furthermore, if necessary, an additional amino acid sequence such as a signal peptide, a marker peptide and a tag amino acid sequence can be connected. If the lignocellulose degradation cofactor and an active fragment thereof contain such an additional amino acid sequence, the additional amino acid sequence is preferably connected so as to form a fusion protein with the lignocellulose degradation cofactor or the like.

The "signal peptide" is a peptide required for transferring a protein biosynthesized in a cell outside the cell. The signal peptide usually has a structure in which an amino acid positively charged such as Lys and Arg is arranged at the N-terminal side of a polypeptide of interest and a highly hydrophobic amino acid sequence such as Ala, Leu, Val, Ile, Val and Phe at the upstream of it. In the present invention, for example, a signal peptide of an extracellular secretory protein can be used.

### 2. Polynucleotide encoding lignocellulose degradation cofactor or active fragment thereof

### 2-1. Outline

A second aspect of the present invention relates to a polynucleotide encoding the lignocellulose degradation cofactor of the first aspect or an active fragment thereof.

### 2-2. Constitution

The polynucleotide of the present invention is constituted of a polynucleotide having a nucleotide sequence encoding the lignocellulose degradation cofactor of the first aspect, more specifically, a polypeptide consisting of the amino acid sequence represented by SEQ ID NO: 1 or 9; a polypeptide consisting of an amino acid sequence obtained by deleting, substituting or adding one or more amino acids in the amino acid sequence represented by SEQ ID NO: 1 or 9; a polypeptide consisting of an amino acid sequence having an amino acid identity of 90% or more with the amino acid sequence represented by SEQ ID NO: 1 or 9; or an active fragment thereof. Specific examples thereof include a polynucleotide consisting of the nucleotide sequence represented by SEQ ID NO: 2 encoding the polypeptide consisting of amino acids sequence represented by SEQ ID NO: 1; or a polynucleotide consisting of the nucleotide sequence represented by SEQ ID NO: 10 encoding the polypeptide consisting of amino acids represented by SEQ ID NO: 9; a polynucleotide consisting of a nucleotide sequence obtained by deleting, substituting or adding one or more nucleotides in the nucleotide sequence represented by SEQ ID NO: 2 or 10; a polynucleotide consisting of a nucleotide sequence having a nucleotide identity of 90% or more, preferably 95% or more, more preferably 97% or more, 98% or more or 99% or more with the nucleotide sequence represented by SEQ ID NO: 2 or 10; or a polynucleotide consisting of a nucleotide sequence hybridizing with a part of the nucleotide sequence complementary with the nucleotide sequence represented by SEQ ID NO: 2 or 10, under stringent conditions, or polynucleotides containing a nucleotide sequence encoding these active fragments. The "nucleotide identity" refers to the ratio (%) of the number of same nucleotide relative to the total number of nucleotides of the polynucleotide consisting of the nucleotide sequence represented by SEQ ID NO: 2 or 10, when two nucleotide sequences are aligned so as to obtain a maximum degree of similarity of nucleotides of them, if necessary, by introducing a gap(s) in either one of the nucleotide sequences. The "stringent conditions" refer to conditions under which a hybrid specific to the nucleotide sequence represented by SEQ ID NO: 2 or 10 is formed and a nonspecific hybrid is not substantially formed. Usually low stringent conditions to high stringent conditions are mentioned; however, high stringent conditions are preferable. The low stringent conditions refer to, for example, washing conditions performed after hybridization in which washing is made with a buffer containing 5 × SSC and 0.1% SDS at 42°C to 50°C. The high stringency conditions refer to, for example, washing conditions performed after hybridization in which washing is made at 65°C and with 0.1 × SSC and 0.1% SDS. Generally, stringency is higher as the salt concentration decreases and the temperature increases. The low salt concentration herein more specifically refers to a concentration of e.g., 15 to 750 mM, preferably 15 to 500 mM, 15 to 300 mM or 15 to 200 mM. The high temperature herein more specifically refers to a temperature of e.g., 50 to 68°C or 55 to 70°C.

### 2-3. Preparation of polynucleotide

The polynucleotide of the present invention can be obtained by, for example, southern blotting or PCR from a cDNA library of an appropriate species by using a part of the nucleotide sequence represented by SEQ ID NO: 2 or 10 or a complementary sequence therewith as a probe or a primer; preferably a cDNA library or genomic library derived from a plurality of microorganisms (including bacterium, filamentous fungus, protozoan, etc.) prepared by a metagenome analysis method. These methods are techniques known in the art and may be carried out in accordance with the method described in general protocol in the art, for example, Green, M. R. and Sambrook, J., 2012, Molecular Cloning: A Laboratory Manual Fourth Ed., Cold Spring Harbor Laboratory Press, Cold Spring Harbor, New York.

### 3. Expression vector

### 3-1. Outline

A third aspect of the present invention relates to an expression vector containing the polynucleotide of the second aspect expressibly. In the expression vector of the present invention, the expression of the polynucleotide described as the second aspect can be easily controlled. Furthermore, by introducing the vector into a host, a transformant according to a fifth aspect (described later) can be obtained as an active ingredient of a cellulase activator (described later).

### 3-2. Constitution

The "expression vector" generally refers to a vector capable of controlling expression of a gene encoded within the vector. The term "expressibly" refers to the state in which the polynucleotide contained in the expression vector can be transcribed in the predetermined condition of a host. For example, the state where the polynucleotide is arranged under the control of a host-specific promoter and terminator contained in the expression vector, corresponds to this.

The core part of the expression vector of the present invention, i.e., a mother nucleus vector, is not particularly limited; however, a plasmid or virus replicable in a host cell is preferable. For example, if the host is a filamentous fungus, a plant plasmid of a binary vector, by which a gene of interest can be introduced into a plant cell via an *agrobacterium,* such as a pBI series plasmid (for example, pBI121, pBI101, pBI101.2, pBI101.3, pBIG2113) or a pRI series plasmid; and a plant virus such as a cauliflower mosaic virus (CaMV), a kidney bean golden mosaic virus (BGMV) and a tobacco mosaic virus (TMV), can be used. Furthermore, a shuttle vector between *Escherichia coli* and a plant such as pUC18 series, pUC19 series and pUC9 series plasmids also can be used. These expression vectors are commercially available from life science manufacturers and these commercially available products can be used.

The expression vector may contain regulatory regions such as a promoter or a terminator, and/or a marker region such as a marker/selection marker gene. Other than these, e.g., an enhancer, a poly adenylation signal, 5'-UTR (untranslated region) sequence, a marker or selection marker gene, a multi-cloning site and an origin of replication can be contained. The types of these are not particularly limited as long as they can achieve their functions in a host cell. These known in the art may be appropriately selected in accordance with the host to which the vector is to be introduced.

As the regulatory regions, regulatory regions that are operable within a host cell are used. In the case of the expression vector of the present invention, regulatory regions that are operable within a plant cell and a filamentous fungal cell are preferable.

Of the regulatory regions, a promoter may be used in accordance with the desired expression pattern; for example, an overexpressing promoter, a constitutively active promoter, a site-specific promoter, a stage-specific promoter and/or an inducible promoter can be used. Specific examples of an overexpressing constitutively active promoter include a cauliflower mosaic virus (CaMV)-derived 35S promoter, Ti plasmid-derived promoter Pnos for a nopaline synthase gene, a corn-derived ubiquitin promoter, a rice-derived actin promoter and tobacco-derived PR protein promoter. Furthermore, a small subunit (Rubisco ssu) promoter for ribulose diphosphate carboxylase, a histone promoter, a cysteine-protease promoter, a metallothionein (MTT)1 promoter, a MTT2 promoter and the like can be used.

Examples of the terminator include, a terminator for a nopaline synthase (NOS) gene, a terminator for an octopine synthase (OCS) gene, a CaMV 35S terminator, a 3' terminator for *E*. *coli* lipopolyprotein lpp, a trp operon terminator, an amyB terminator, and a terminator for an ADH1 gene. The terminator is not particularly limited as long as it has a sequence which can terminate transcription of a gene transribed by a promoter as mentioned.

Examples of the enhancer include a CMV enhancer region including a sequence upstream within the CaMV 35S promoter. The enhancer is not particularly limited as long as it can enhance the expression efficiency of a polynucleotide encoding the lignocellulose degradation cofactor or the like of the first aspect.

Examples of the marker/selection marker gene include a drug resistance gene (for example, a tetracycline resistance gene, an ampicillin resistance gene, a kanamycin resistance gene, a hygromycin resistance gene, a spectinomycin resistance gene, a chloramphenicol resistance gene or a neomycin resistance gene); a nutrient gene (for example, a synthetic gene for leucine, uracil, adenine, histidine, lysine or tryptophan); a fluorescent or luminescent reporter gene (for example, gene for luciferase, β-galactosidase, β-glucuronidase (GUS) or green fluorescent protein (GFP)); an enzyme gene (for example, gene for neomycin phosphotransferase II (NPT II) or dihydrofolate reductase); and an enzyme gene such as blasticidin S resistance gene.

### 3-3. Preparation of expression vector

A method for preparing an expression vector of the present invention will be exemplified below. A fundamental protocol for e.g., the gene manipulation may be carried out in accordance with a method known in the art, for example, a method described in Green, M. R. and Sambrook, J., 2012 (vide supra).

### (1) Cloning of lignocellulose degradation cofactor gene

First, the gene encoding the lignocellulose degradation cofactor described as the first aspect to be introduced in an expression vector is cloned. This gene can be obtained, for example, by using a contract-based synthesizing service provided by a biomarker, furthermore, by completely and artificially synthesizing a gene encoding the lignocellulose degradation cofactor based on the nucleotide sequence represented by SEQ ID NO: 2 or 10. Alternatively, the gene may be prepared from cDNA library(s) prepared from *Neotermes koshunensis* and/or *Coptotermes formosanus* containing intestinal bacteria by e.g., a nucleic acid amplification method such as PCR or a plaque hybridization method based on the nucleotide sequence represented by SEQ ID NO: 2 or 10. The cDNA library(s) of *Neotermes koshunensis* or *Coptotermes formosanus* containing intestinal bacteria is prepared in accordance with the method described in, for example, Green, M. R. and Sambrook, J., 2012 (vide supra) after *Neotermes koshunensis* or *Coptotermes formosanus* is obtained. *Neotermes koshunensis* is distributed in Okinawa prefecture in Japan and can be easily collected from fallen trees and deadwoods in the forest. *Coptotermes formosanus* is distributed on the pacific side of Kyushu, Shikoku and Honshu in Japan, and can be relatively easily collected from not only forests but also old wooden houses. In the case where a gene of interest is prepared from a cDNA library by a nucleic acid amplification method, oligonucleotides serving as a primer pair are chemically synthesized based on the nucleotide sequence represented by SEQ ID NO: 2 or 10 and then amplification may be carried out using the primer pair to prepare the gene from the cDNA library. At this time, it is convenient to add an appropriate restriction site designed for vector insertion at the 5' end side of each of the primers. As the DNA polymerase for use in nucleic acid amplification, an enzyme high in fidelity (accuracy) having a 3'-5' exonuclease activity such as pfu polymerase is preferably used. As to specific conditions for nucleic acid amplification, for example, a method described in Innis M. et al (Ed.), (1990) Academic Press, PCR Protocols: A Guide to Methods and Applications, may be referred to. The isolated gene of interest is, if necessary, inserted in a suitable plasmid and cloned in a host microorganism such as *E. coli,* and then, a full-length nucleotide sequence thereof is preferably checked based on a known technique. In the case where a gene of interest is isolated from a cDNA library in accordance with a plaque hybridization method, an appropriate region of the nucleotide sequence represented by SEQ ID NO: 2 or 10 is selected and an oligonucleotide having the nucleotide sequence of the region is chemically synthesized. Then, using the oligonucleotide as a probe, the gene of interest may be isolated from the cDNA library by using a known plaque hybridization method. As to the details of the plaque hybridization method, Green, M. R. and Sambrook, J., 2012 (vide supra) may be referred to.

### (2) Preparation of plasmid expression vector

Next, a plasmid expression vector is prepared. This may be prepared by inserting the gene encoding the lignocellulose degradation cofactor cloned in the above (1), in other words, the polynucleotide of the second aspect, into a predetermined site in the mother nucleus vector moiety of a desired expression vector.

To describe more specifically, for example, both ends of the polynucleotide of the second aspect are cleaved with an appropriate restriction enzyme; whereas, the mother nucleus vector moiety of the expression vector is cleaved at the corresponding restriction enzyme sites. If an expression vector has a multi-cloning site, it is convenient to employ such a vector. Subsequently, the ends of both nucleic acids are connected by e.g., ligase, the gene encoding the lignocellulose degradation cofactor is inserted into the expression vector. At this time, attention must be paid to inserting the gene to be placed under the control of the promoter into the expression vector. Incidentally, the expression vector may be prepared by using a commercially available gene expression system or kit.

### (3) Preparation of virus expression vector

Fundamental protocol may be carried out in accordance with the aforementioned method for a plasmid expression vector. First, a viral genome is prepared by a method known in the art and then inserted into an appropriate cloning vector (for example, *E. coli-*derived pBI series, pPZP series, pSMA series, pUC series, pBR series, pBluescript series plasmids) to obtain a recombinant. Subsequently, the gene encoding the lignocellulose degradation cofactor cloned in the above (1) is inserted into a predetermined site within the viral genome to be contained in a recombinant to obtain a clone. Subsequently, the viral genomic region may be excised out from the recombinant with a restriction enzyme. In this manner, a virus expression vector of interest can be obtained.

### 4. Transformant or progeny thereof

### 4-1. Outline

A fourth aspect of the present invention relates to a transformant obtained by introducing the expression vector of the third aspect into a host cell or a progeny thereof (hereinafter sometimes referred to as the "transformant or the like"). The transformant or the like according to the present invention allows production of the lignocellulose degradation cofactor described as the first aspect by culture and expression induction treatment thereof.

### 4-2. Constitution

In the specification, the "transformant" is a host transformed by introduction of the expression vector of the third aspect and refers to a transformant of the first generation. In the transformant of this aspect, the expression vector of the third aspect is present independently in the host cell or integrated into the genome thereof. The number of types of expression vectors present in a single transformant cell may be usually one; however, a plurality of types of expression vectors may be present.

In the specification, the "host" refers to an organism, cell, or tissue to which the expression vector of the third aspect is to be introduced and in which the polynucleotide of the second aspect contained in the expression vector can be expressed to produce the polypeptide of the first aspect, in other words, the lignocellulose degradation cofactor. The host to be the transformant of this aspect is not particularly limited as long as the expression vector introduced therein can be replicated and can express the polypeptide of the second aspect contained therein. For example, any one of a fungus, a bacterium and a plant may be used. Examples of the fungus include a filamentous fungus, a basidiomycete and a yeast. Examples of the filamentous fungus (mold) include fungi belonging to the genera *Trichoderma, Aspergillus, Penicillium, Neurospora, Fuzarium* and *Streptomyces.* Examples of the yeast include a budding yeast (*Saccharomyces cerevisiae*), a fission yeast (*Schizosaccharomyces pombe*) and a methanol utilizing yeast (*Pichia pastoris*). Examples of the bacteria include *E. coli, Bacillus, Pseudomonas* and *Actinomyces.* Examples of the plant include a moss, a fern, an angiosperm and a gymnosperm. The host is preferably a filamentous fungus and more preferably a fungus of the genus *Trichoderma.*

In the specification, the "progeny of the transformant" refers to a second-generation transformant or later, which is obtained by asexual reproduction or sexual reproduction of the transformant (first generation) and keeping the expression vector of the third aspect. For example, in the case of a unicellular microorganism, a new cell (clone body), which generates from the first-generation transformant or later by e.g., cell division or budding, correspond to this. In the case where a host transformed is a filamentous fungus, a fungus body regenerated from a part of the hyphae taken from the fungus body of the first-generation transformant or later or a seedling (including a spore) of the first-generation transformant or later, correspond to this.

### 4-3. Preparation of transformant

A method for preparing the transformant of the present invention by introducing the expression vector of the third aspect in a host may be carried out in accordance with a transformation method known in the art.

If the host is a bacterium, a heat shock method, a calcium ion method (for example, calcium phosphate method) and an electroporation method are exemplified. If the host is a yeast, a lithium method or an electroporation method are exemplified. These techniques are all known in the art and described in various literatures including Green, M. R. and Sambrook, J., 2012 (vide supra) and thus these literatures may serve as references for details of the methods.

In the case where the host is a filamentous fungus or a plant and the expression vector is a plasmid vector, any method known in the art may be appropriately used as the transformation method. As a preferable transformation method, an *agrobacterium* method, a protoplast method, a particle gun method or the like can be used.

The *agrobacterium* method is a transformation method using, as a transformation factor, bacterium belonging to the genus *Agrobacterium* (for example, *A*. *tumefaciens* and *A*. *rhizogenes*) and a Ti plasmid derived therefrom; and a DNA of interest (herein, the expression vector of the third aspect) can be introduced in the genome DNA of a host filamentous fungus or a host plant.

The protoplast method is a method of introducing a DNA of interest into a filamentous fungus or a plant cell by using a cell (protoplast) prepared by removing the cell wall with an enzymatic treatment using e.g., cellulase. This method can be further subdivided according to the manner of introducing DNA into e.g., an electroporation method, a microinjection method and a polyethylene glycol method. The electroporation method is a method of introducing DNA into a protoplast by applying an electric pulse to a mixture of the protoplast and the DNA of interest. The microinjection method is a method of directly introducing a DNA of interest into a protoplast by use of a microneedle under a microscope. The polyethylene glycol method is a method of introducing a DNA of interest into a protoplast by application of polyethylene glycol.

The particle gun method is a method of introducing a DNA of interest into a filamentous fungus or plant host cell by attaching the DNA of interest to a fine particle of e.g., gold or tungsten and injecting the particle with the help of a high pressure gas. In this method, a transformed cell having a gene of interest integrated in the genome DNA of the host can be obtained. A transformed cell is selected usually based on a product of a marker/selection marker gene within the expression vector. Other than this, whether the expression vector is introduced or not can be checked by e.g., a PCR method, a Southern hybridization method, a Northern hybridization method or an *in-situ* hybridization.

The aforementioned methods are all known in the art. For the details, appropriate protocols for plant genetic engineering may be referred to.

In the case where the expression vector is a virus expression vector (for example, CaMV, BGMV, TMV as mentioned above), a transformed cell can be obtained by infecting a host cell with the expression vector described as the third aspect. As to the details of the gene introduction method using such a virus expression vector, the method of Hohn et al. (Molecular Biology of Plant Tumors, Academic Press, New York, 1982, pp 549) may be referred to.

### 4-4. Method for obtaining progeny

A method for obtaining a progeny from a transformant of the present invention may be carried out in accordance with a method usually used for obtaining a progeny in the species to which the host to be the transformant belongs to. If the host to be a transformant is, for example, a bacterium or a yeast, a progeny can be easily obtained by culturing the transformant in an appropriate known medium. As to the composition of the medium, for example, Green, M. R. and Sambrook, J., 2012 (mentioned above) may be referred to. In the case where the host to be a transformant is a filamentous fungus or a plant, a progeny can be obtained by usually cultivating a spore or a seed, a vegetative propagation organ, or a fungus body or a part of a plant body.

### 4-5. Effect

The lignocellulose degradation cofactor or the like described as the first aspect can be produced in appropriate expression conditions by using the transformant or the like of the present invention.

### 5. Cellulase activator

### 5-1. Outline

The fifth aspect of the present invention relates to a cellulase activator. The cellulase activator of the present invention contains the lignocellulose degradation cofactor or the like described as the first aspect as an active ingredient. According to the cellulase activator of the present invention, the object of the present invention, i.e., saccharifying a lignocellulosic biomass at low cost while reducing environmental load can be attained.

### 5-2. Constitution

### (1) Active ingredient

The cellulase activator of the present invention contains at least one of the lignocellulose degradation cofactor or the like described as the first aspect as an active ingredient. In the case where a plurality of types of the lignocellulose degradation cofactor or the like are contained, the combination of them is not particularly limited. Any combination may be employed as long as the combination does not suppress the activity of the other cofactor(s) or the like and preferably the combination brings a synergetic effect on enhancement of the cellulase activity.

The content of the active ingredient per predetermined amount of the cellulase activator of the present invention varies depending upon various conditions such as a type of lignocellulose biomass, a dosage form of the cellulase activator, an application method and a purpose of application. Usually, the content is not particularly limited as long as the active ingredient, i.e., the lignocellulose degradation cofactor or the like, is not inactivated and denatured in the cellulase activator of the present invention, and can enhance the activity of cellulase co-present in a lignocellulosic biomass during application. The content may be appropriately determined in consideration of proper conditions.

The cellulase activator of the present invention may be a in a form of concentrate, which is applied by diluting it with an appropriate solvent to 10 to 1000 fold. In this case, the active ingredient may be contained in a concentration of 10 to 1000 fold as large as a normal dose in the cellulase activator liquid concentrate.

### (2) Solvent and/or carrier

The cellulase activator of the present invention can contain a solvent and/or a carrier in addition to the above active ingredient, i.e., the lignocellulose degradation cofactor or the like.

In the specification, the "solvent" and "carrier" has a function of maintaining the activity of the active ingredient in the cellulase activator and facilitating application of the active ingredient to lignocellulose and may have a function of controlling the cellulase activity enhancement action of the active ingredient by adjusting reaction conditions such as pH at the time of application to lignocellulose. In principle, a solvent and a carrier are legally approved for use in a lignocellulosic biomass and are desirably substances having no or less harmful effect on the environment such as water pollution and/or animals, particularly humans.

Examples of the "solvent" include water or aqueous solutions except water. Examples of the aqueous solutions include a buffer such as a phosphate buffer, physiological saline and a liquid medium. In particular, the liquid medium is convenient because the culture supernatant of the transformant or the like described as the fourth aspect can be directly used as the cellulase activator of the present invention, with the result that e.g., a purification step of the lignocellulose degradation cofactor or the like is not required and the cellulase activator can be produced simply and at a low cost. The liquid medium is not particularly limited as long as it can culture the transformant or the like described as the fourth aspect. The type and composition of the medium may be acceptable as long as they are known in the art and used in accordance with the type of host to be a transformant. For example, if the host is a fungus or an algae, e.g., YPD medium (Yeast extract 20 g/L, Proteose peptone 10 g/L, D-glucose 5 g/L); King B medium (2% [w/v] peptone, 1% [w/v] glycerin, 0.15% [w/v] dipotassium hydrogen phosphate, 0.15% [w/v] magnesium sulfate, heptahydrate); PSB medium (200 g/L potato decoction, 0.5% [w/v] sucrose); and C medium (Ca(NO₃)₂·4H₂O 150 mg/L, KNO₃ 100 mg/L, β-Na₂ glycerophosphate·5H₂O 50 mg/L, MgSO₄·7H₂O 40 mg/L, Vitamin B₁₂ 0.1 µg/L, Biotin 0.1 µg/L, Thiamine HCl 10 µg/L, PIV metals 3mL/L, Tris (hydroxymethyl) aminomethane 500 mg/L, pH 7.5) can be used. The mediums to be used for individual hosts and the compositions thereof are described in various protocols, for example, Green, M. R. and Sambrook, J., 2012(vide supra) and these protocols may be referred to.

Examples of the "carrier" include a ground natural mineral (for example, kaolin, clay, talc and chalk), a ground synthetic mineral (for example, highly dispersible silica and silicate), an emulsifier (for example, nonionic emulsifiers and anionic emulsifiers), a dispersant (lignosulfite waste liquors and methylcellulose), a fluidizing agent/modifier (for example, silicate, stearate or polyethylene glycol), a pH modifier, a surfactant, a tonicity agent, a solubilizer, a suspending agent, a diluent and a stabilizer. The cellulase activator may contain at least one of these carriers.

### (3) Other components

The cellulase activator of the present invention can contain, if necessary, other components in addition to the active ingredient and solvent and/or carrier. For example, cellulase responsible for a main reaction in saccharifying lignocellulose is mentioned.

In the specification, the "cellulase" refers to a collective term of enzymes for hydrolyzing a β-1,4-glucan or a β-D-glucosidic bond of cellulose to produce cello-oligosaccharide, cellobiose and β-D-glucose. Three types of cellulases are known: endoglucanase, exoglucanase and β-glucosidase, depending upon the action mode. Endoglucanase mainly acts on an amorphous moiety of a cellulose fiber and cleaves an internal cellulose sugar chain. Exoglucanase acts on a crystalline cellulose to degrade the ends of a cellulose sugar chain to produce cellobiose. In contrast, β-glucosidase separates a final product, i.e., β-D-glucose, from e.g., cellobiose and/or cello-oligosaccharide produced by the actions of endoglucanase and/or exoglucanase. In the specification, any type of cellulase may be used. The type of cellulase is not specified.

### 5-3. Dosage form

The dosage form of the cellulase activator of the present invention may be in any state as long as it is applicable to a lignocellulosic biomass. For example, a liquid formulation or a solid formulation can be used. Examples of the liquid formulation include a solution, an oily dispersion, an emulsion and a suspension, which are obtained by suspending an active ingredient in an appropriate solvent. The solid formulation is not particularly limited as long as the active ingredient can act on cellulase co-present in a lignocellulosic biomass. Examples thereof include a dusting powder, a powder, a paste, a pellet and a gel.

### 5-4. Method for producing cellulase activator

The method for producing the cellulase activator of the present invention comprises an active ingredient preparation step and formulation step.

### (1) Active ingredient preparation step

The "active ingredient preparation step" is a step of preparing the active ingredient of the cellulase activator, i.e., the lignocellulose degradation cofactor or the like. A method for preparing the lignocellulose degradation cofactor or the like described as the first aspect is not particularly limited. Herein, as an example, a method for preparing the active ingredient by using the transformant or the like described as the fourth aspect will be described. In this method, the active ingredient preparation step further comprises a culturing step, an expression induction step and a recovery step.

The "culturing step" is a step of culturing the transformant or the like described as the fourth aspect in appropriate culture conditions. The transformant or the like described as the fourth aspect has the expression vector described as the third aspect. The expression vector contains the active ingredient, i.e., the lignocellulose degradation cofactor or the like, expressibly. Accordingly, by culturing the transformant or the like described as the fourth aspect and carrying out an expression induction treatment in the next expression induction step, the transformant or the like can produce a lignocellulose degradation cofactor or the like of interest from the expression vector contained therein. A method for culturing the transformant or the like described as the fourth aspect may be carried out in accordance with a culture method usually used for a host to be a transformant. The medium for use in culture is not particularly limited as long as it contains e.g., a carbon source, a nitrogen source and inorganic salts that the host can utilize, grow and proliferate therein. Either one of a natural medium and a synthetic medium may be used. Specific culture method for the transformant or the like may be carried out in accordance with a culture method for the host known in the art. Since these methods are described in various protocols, for example, Green, M. R. and Sambrook, J., 2012 (vide supra), these protocols may be referred to. The culture temperature and culture time may be appropriately determined in accordance with the host and are not particularly limited; however, culture may be usually carried out at a temperature of 10 to 45°C, 15 to 40°C or 18 to 37°C for one hour to 14 days, 10 hours to 10 days or 15 hours to 7 days. If necessary, ventilation, irradiation and/or stirring can be made.

The "expression induction step" is a step of inducing expression of a polynucleotide encoding the lignocellulose degradation cofactor or the like in the expression vector contained in the transformant or the like by subjecting the transformant or the like cultured to a predetermined expression induction treatment. The expression induction method varies depending upon e.g., the promoter contained in the expression vector, an induction treatment suitable for the expression vector contained in the transformant or the like may be carried out. Induction of the lignocellulose degradation cofactor or the like is attained usually by adding an appropriate amount of inducer to a medium. Note that, if the expression vector contained in the transformant or the like constantly expresses the lignocellulose degradation cofactor or the like, even if an induction treatment is not specifically applied, this step can be simultaneously attained in the culturing step.

The "recovery step" is a step of recovering the lignocellulose degradation cofactor or the like induced in the expression induction step from the transformant or the like or its culture liquid. In the case where the lignocellulose degradation cofactor or the like expressed is produced within the cells of the transformant, the cells are recovered by e.g., centrifugation, broken by a cell homogenizer such as sonicator and subjected to e.g., cell extract or centrifugation to obtain a cell debris-free extraction supernatant. In this manner, the active ingredient, the lignocellulose degradation cofactor or the like, contained in the cell extract can be prepared. The cell extract or extraction supernatant obtained is, if necessary, further subjected to a known protein purification method, for example, solvent extraction, salting out, solvent precipitation, dialysis, ultrafiltration, gel electrophoresis, ammonium sulfate precipitation, gel filtration chromatography, ion exchange chromatography, reverse phase chromatography and affinity chromatography. These may be used alone or appropriately in combination to separate the lignocellulose degradation cofactor or the like from the culture. In this case, the lignocellulose degradation cofactor or the like purified is prepared as the active ingredient. As to details of the protein purification methods, the method of Hayashi et al. (Hayashi et al., 1996, Phytochemistry, 42: 665-666) and the method of Noguchi et al. (Noguchi et al., 2007, J. Biol. Chem., 282: 23581-23590) may be referred to. In the case where the lignocellulose degradation cofactor or the like expressed is secreted outside the transformant cell, a culture liquid or a culture supernatant, which is obtained by centrifugally removing the transformant or the like from the culture liquid, is obtained. In this manner, the lignocellulose degradation cofactor or the like as the active ingredient contained in the culture liquid or culture supernatant can be prepared. If necessary, from the culture liquid or culture supernatant, the lignocellulose degradation cofactor or the like may be prepared by separating and purifying it by a known protein purification method.

### (2) Formulation step

The "formulation step" is a step of formulating the cellulase activator containing the lignocellulose degradation cofactor or the like as an active ingredient prepared in the active ingredient preparation step.

In this step, the active ingredient, the lignocellulose degradation cofactor or the like, prepared in the active ingredient preparation step is mixed with a solvent and/or carrier as mentioned above and, if necessary, other components such as cellulase, is added to formulate the cellulase activator of the present invention. At this time, the content of the lignocellulose degradation cofactor or the like may be appropriately determined in consideration of various conditions such as a type of lignocellulose biomass, dosage form, application method and application purpose of the cellulase activator, as described above.

The cell extract and the culture liquid in the case where the lignocellulose degradation cofactor or the like is secreted outside a transformant cell obtained by the recovery step can be directly used as the cellulase activator without applying a special treatment in this step. Particularly, in the method of directly using the culture liquid, in which the transformant or the like described as the fourth aspect is cultured, as the cellulase activator, the cellulase activator of this aspect can be produced from the transformant or the like simply in fewer steps and at low cost.

### 6. Method for saccharifying lignocellulosic biomass

### 6-1. Outline

The sixth aspect of the present invention relates to a method for saccharifying a lignocellulosic biomass. This method is a saccharification method in which a lignocellulosic biomass is hydrolyzed with cellulase to produce reducing sugar β-D-glucose as a degradation product of lignocellulose. In this method, the activity of cellulase can be enhanced by adding the cellulase activator described as the fifth aspect before or during the degradation with cellulase.

### 6-2. Method

The saccharification method of the present invention comprises a cellulase mixing step, a cellulase activator mixing step and a reaction step as essential steps. As an optional step, a lignocellulose pretreatment step can be included. The individual steps will be described below.

### (1) Lignocellulose pretreatment step

The "lignocellulose pretreatment step" is a step of previously treating the lignocellulosic biomass to be used in the saccharification method of the present invention to obtain the state of the biomass to be easily hydrolyzed. This step is an optional step and may be carried out as necessary. This step, if carried out, should be performed prior to the cellulase mixing step and cellulase activator mixing step (described later).

The lignocellulose pretreatment comprises a fragmentation treatment and/or a hydrothermal treatment.

In the present invention, the "fragmentation treatment " is a treatment for fragmenting a lignocellulosic biomass. The "fragmentation" refers to cutting and/or grinding a lignocellulosic biomass into pieces in the form of chip, granule or powder. Fragmentation into powdery pieces is preferable. Fragmentation of a lignocellulosic biomass may be carried out by using a known apparatus such as a (converge) mill, a wood chipper and a straw cutter. Owing to the fragmentation, the interior tissue of lignocellulose is exposed and the ratio of the surface area can be increased. As a result, the contact ratio of lignocellulose and cellulase is increased and the hydrolysis efficiency of lignocellulose can be enhanced.

In the present invention, the "hydrothermal treatment" refers to a method for treating a lignocellulosic biomass with subcritical water under high temperature and high pressure conditions. As the solvent for the hydrothermal treatment, water and a phosphoric acid aqueous solution having a function of accelerating mono-saccharification of a polysaccharide can be used. In the case of the phosphoric acid aqueous solution, the concentration of a phosphoric acid may fall within the range of 0.1 to 5 wt% and preferably 0.2 to 4 wt%. The pressure to be applied, i.e., initial pressure, may fall within the range of 1 to 5 MPa (10 to 50 atmospheric pressure) and preferably 1.5 to 3 MPa. The treatment is preferably performed at a temperature within the range of 140 to 280°C and preferably 140 to 260°C. This is because hemicellulose of lignocellulose is usually hydrolyzed at a temperature of 140 to 260°C. The treatment may be performed within the time range of 5 minutes to 2 hours and preferably 10 minutes to one hour. Note that the heating time for increasing the temperature up to the treatment temperature is not included. Application of pressure is carried out within a pressure-resistant sealed container. The air in the container is preferably replaced with a low reactive inert gas before the main treatment. Examples of the inert gas other than nitrogen include a rare gas such as helium, neon, argon and xenon. The inert gas is preferably nitrogen or argon.

### (2) Cellulase mixing step

The "cellulase mixing step" is a step of mixing a lignocellulosic biomass and cellulase. This step is an essential step in the saccharification method of the present invention.

The lignocellulosic biomass to be used in this step is preferably a suspension prepared by mixing the biomass and an appropriate solvent. This is because the saccharification method of the present invention is an enzymatic degradation method in which the reaction proceeds in a liquid. Note that, in this step, dry-state lignocellulosic biomass and a solvent may be mixed in a reaction tank to obtain a suspension. The solvent is preferably water or a buffer having a salt concentration and pH adjusted to fall within optimum ranges of the cellulase reaction.

The cellulase to be added in this step may be derived from any species as long as it has a cellulose hydrolytic activity. For example, cellulase may be derived from any one of a bacterium, a fungus, a slime mold, an insect such as *Isoptera* and *Cryptocercus* and a protozoan. For example, cellulase (endoglucanase) derived from *Trichoderma reesei* can be used. The endoglucanase to be used in this step is not necessary purified; for example, an unpurified (crude) solution containing cellulase like a culture supernatant of *Trichoderma reesei* can be used.

The amount of cellulase added relative to a lignocellulosic biomass varies depending upon whether a pretreatment is applied to the lignocellulose or not, and the type of cellulase and thus, the amount to be added may be appropriately set in consideration of individual conditions. Usually, the amount to be added may be, for example, 1 to 10 mg per lignocellulose (1 g). In the case where an unpurified solution containing cellulase, like a culture supernatant of an organism producing cellulase, is used in this step, 1 to 10 mL of the unpurified solution may be added per lignocellulose (1 g).

In this step, as long as a lignocellulosic biomass and cellulase can be mixed in the reaction tank, the order of mixing them is not particularly limited. To be more specific, cellulase may be added to a lignocellulosic biomass placed in a reaction tank; conversely, a lignocellulosic biomass may be added to cellulase placed in the reaction tank. Alternatively, they can be simultaneously put in the reaction tank.

As long as a lignocellulosic biomass and cellulase can be sufficiently mixed, the mixing method and means are not limited. For example, a method using a stirring device such as a stirring rod and a method inverting, rotating or vibrating a reaction tank may be mentioned.

### (3) Cellulase activator mixing step

The "cellulase activator mixing step" is a step of mixing the cellulase activator described as the fifth aspect with a lignocellulosic biomass. This step is an essential step in the saccharification method of the present invention. The order of this step and the cellulase mixing step is not limited. The cellulase mixing step can be carried out before, after or simultaneously with this step.

The amount of cellulase activator added relative to a lignocellulosic biomass varies depending upon whether the lignocellulose pretreatment is applied or not and the types of cellulase and cellulase activator and thus, the amount to be added may be appropriately set in consideration of individual conditions. In the case where a culture supernatant of the transformant described as the fourth aspect is used as the cellulase activator, the amount to be added may be, for example, 0.5 to 10 mg per lignocellulose (1 g).

The mixing method may be carried out in accordance with the method described in the cellulase mixing step.

### (4) Reaction step

The "reaction step" is a step of hydrolyzing lignocellulose by bringing a lignocellulosic biomass and cellulase into contact with each other in the presence of the cellulase activator. This step is an essential step in the saccharification method of the present invention. This step is carried out after the cellulase mixing step and the cellulase activator mixing step.

In the reaction tank, lignocellulose is hydrolyzed with cellulase by bringing a lignocellulosic biomass and cellulase into contact with each other in the presence of the cellulase activator. Since the activity of cellulase can be enhanced by the function of the cellulase activator, an efficient saccharification reaction of lignocellulose can proceed.

In this step, the reaction conditions vary depending upon whether the pretreatment is applied to a lignocellulosic biomass or not and the amount of lignocellulosic biomass, the types and amount of cellulase and cellulase activator to be added; however, the reaction conditions are not particularly limited as long as cellulase can maintain high activity in the presence of the cellulase activator and may be appropriately set in consideration of individual conditions. Usually, incubation may be carried out in the range of pH 4 to pH 6, preferably pH 4.5 to pH 5.5, at a temperature of 40°C to 60°C, preferably 45°C to 55°C and for 24 hours to 150 hours, preferably 48 hours to 120 hours.

In the reaction step, a mixed reaction solution may be stirred or allowed to stand still.

After the reaction, reaction residue is removed by e.g., centrifugation and/or filtration. In this manner, a saccharified solution of β-D-glucose derived from a lignocellulosic biomass can be obtained.

### Examples

### <Example 1: Isolation of lignocellulose degradation cofactor>

(Purpose) A non-cellulase biomass degradation cofactor is isolated from flagellate symbiotically living in the digestive tracts of *Neotermes koshunensis, Coptotermes formosanus* and *Cryptocercus* by metatranscriptomic analysis.

### (Method)

*Neotermes koshunensis* and *Coptotermes formosanus* living in Iriomotejima of the Okinawa prefecture were obtained. A full-length cDNA library was prepared from the total mRNA of intestinal protoctista of each of them based on the oligo-capping method (Maruyama, K., Sugano., Gene 138: 171-174, 1994). The nucleotide sequence of mRNA in the library (metatranscriptome library) obtained was exhaustively analyzed. Analysis was carried out using the nucleotide sequence obtained by BLAST. Furthermore, 2636A49 clone having the nucleotide sequences of hemicellulose and cellulose and carbohydrate binding module which were presumed to be lignin degradation related factors; and 1028A66 clone having the nucleotide sequence of xylanase/chitin deacetylase based on the information of CAZy (http: //www.cazy.org/), were isolated as the lignocellulose degradation cofactors.

### <Example 2: Preparation of cellulase activator>

### (Purpose)

The cellulase activators of the present invention having the lignocellulose degradation cofactors isolated in Example 1 as an active ingredient are prepared.

### (Method and results)

### (1) Construction of expression vector

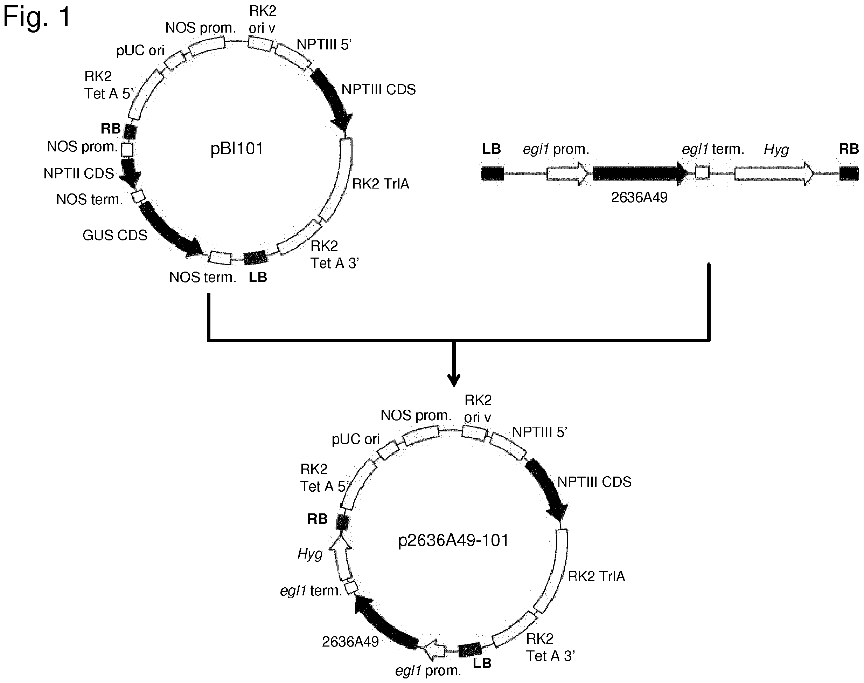

Using a binary vector, pBI101 for plant transformation as shown in Figure 1, as a mother nucleus vector, expression vectors p2636A49-101 and p1028A66-101 for transforming *Trichoderma* in accordance with the *agrobacterium* method were constructed.

Using a primer set consisting of RB-F represented by SEQ ID NO: 3 and LB-R represented by SEQ ID NO: 4 and pBI101 as a template, Inverse PCR was carried out to prepare an expression vector. In the PCR reaction, KOD-plus-DNA polymerase (TOYOBO) was used.

PCR amplification was carried out by using an expression cassette, which contains a nucleotide sequence (SEQ ID NO: 7) of about 1 kb present upstream from the initiation codon of egl1 gene including a promoter region of *Trichoderma-derived* cellulase; 2636A49 cDNA sequence represented by SEQ ID NO: 2; a nucleotide sequence (SEQ ID NO: 8) of about 1 kb present downstream from the termination codon of egl1 including a terminator region of *Trichoderma-derived* cellulase; and a marker sequence (hygromycin resistance gene: Hyg), and by using a primer set consisting of 2636A49-F represented by SEQ ID NO: 5 and 2636A49-R represented by SEQ ID NO: 6 to be applied to the both ends of the expression cassette. The PCR amplified fragment obtained was connected to a vector by use of In-fusion kit (Takara Bio Inc.) to construct an expression vector, p2636A49-101 (Figure 1).

Similarly, PCR amplification was carried out by using an expression cassette, which contains a nucleotide sequence (SEQ ID NO: 7) of about 1 kb present upstream from the initiation codon of egl1 gene including a promoter region of *Trichoderma-derived* cellulase; 1028A66 cDNA sequence represented by SEQ ID NO: 9; a nucleotide sequence (SEQ ID NO: 8) of about 1 kb present downstream from the termination codon of egl1 including a terminator region of *Trichoderma-derived* cellulase; and a marker sequence (hygromycin resistance gene: Hyg), and by using a primer set consisting of 1028A66-F represented by SEQ ID NO: 11 and 1028A66-R represented by SEQ ID NO: 12 to be applied to the both ends of the expression cassette. The PCR amplified fragment obtained was connected to a vector by use of In-fusion kit (Takara Bio Inc.) to construct an expression vector, p1028A66.

### (2) Preparation of Trichoderma transformant

The expression vectors, p2636A49-101 and p1028A66-101, constructed in (1) were separately introduced into *Trichoderma* ATCC66589 strain in accordance with the *agrobacterium* method.

To LB medium (1% polypeptone, 0.5% yeast extract, 0.5% NaCl), chloramphenicol (20 µg/mL) and rifampicillin (20 µg/mL) were added. *Agrobacterium* (EHA105 strain) was inoculated in the LB medium and cultured while shaking at 28°C and 250 rpm for 18 hours. The cultured fungus bodies were transferred to a 50-mL centrifuge tube (FALCON) and spun by a centrifuge (TOMY MX-300: TOMY) at 4,000 rpm for 5 minutes at 4°C and the supernatant was removed. The fungus bodies were suspended in ice-cooled sterilized water and then centrifuged again at 4,000 rpm for 5 minutes at 4°C and the supernatant was removed. This operation was repeated twice to wash the fungus bodies. The fungus bodies were further suspended by adding 40 mL of 10% glycerol and then centrifuged at 4,000 rpm for 5 minutes at 4°C and the supernatant was removed. The fungus bodies were suspended in 1 mL of 10% glycerol. Thereafter, the suspension was dispensed in an amount of 50 µL to 1.5 mL Eppendorf tubes and used as competent cells.

To the competent cells thus prepared, 1 µL of p2636A49-101 or p1028A66-101 was added and mixed. To the mixture, pulse was applied by an electroporation system (Micro Pulser; Bio-Rad). Immediately after that, 1 mL of SOC medium (2% polypeptone, 0.5% yeast extract, 50 mM NaCl, 25 mM KCl, 10 mM MgCl₂, 10 mM MgSO₄, 20 mM glucose) was added. The mixture was cultured at 30°C for one hour while shaking and applied onto LB agar medium to which kanamycin was previously added so as to obtain a final concentration of 25 µg/mL. This was subjected to inversion culture at 30°C for 3 days and used as preculture for the following *agrobacterium* method. Note that, as a control for p2636A49-101 and p1028A66-101, pBI101, which is a mother nucleus vector of these expression vectors, was introduced in the same manner.

To LB agar medium to which kanamycin was previously added so as to obtain a final concentration of 25 µg/mL, the pre-cultures of the *agrobacterium* were inoculated and cultured at 28°C and 250 rpm for 18 hours. The cultured fungus bodies were transferred to a 50-mL centrifuge tube (FALCON) and spun by a centrifuge (TOMY; TOMY MX-300) at 4,000 rpm for 5 minutes at 4°C. After the supernatant was removed, the fungus bodies were suspended in IM medium and centrifuged at 4,000 rpm for 5 minutes at 4°C to wash the fungus bodies. The fungus bodies washed were suspended in IM medium so as to obtain OD₆₀₀ of about 0.2. To the suspension, 200 µM Acetosyringone (Sigma-Aldrich) was added and the suspension mixture was cultured while shaking at 28°C and 250 rpm for 8 hours until OD₆₀₀ of about 1.0 was obtained. This was used as the *agrobacterium* culture liquid.

Subsequently, a spore suspension (1 × 10⁶/mL) of *Trichoderma* ATCC66589 strain and the *agrobacterium* culture liquid prepared were mixed in equal amounts and applied onto IM agar medium on the bed of cellophane membrane. The *Trichoderma* ATCC66589 strain is a strain prepared by introducing β-glucosidase of *Aspergillus aculeatus* to *Trichoderma.* After co-culture was carried out at 24°C for 3 days, the cellophane membrane was transferred to PDA medium (Difco), to which hygromycin was added so as to obtain a final concentration of 100 µg/mL and 200 µM cefotaxime was added, and cultured at 28°C for 3 to 5 days. In this manner, removal of *agrobacterium* and screening of a *Trichoderma* transformant were carried out.

### (3) Preparation of cellulase activator

Spores (1 × 10⁶) of the *Trichoderma* transformant screened were inoculated in a *Trichoderma* expression medium (1% Avicel, 0.14%(NH₄)₂SO₄, 0.2% KH₂PO₄, 0.03% CaCl₂·2H₂O, 0.03% MgSO₄·7H₂O, 0.1% polypeptone, 0.05% yeast extract, 0.1% Tween 80, 0.1% Trace element, final concentration 50 mM citric acid buffer, pH4.0/Trace element: 6mg H₃BO₃, 26mg (NH₄)₆MO₇O₂₄·4H₂O, 100mg FeCl₃·6H₂O, 40mg CuSO₄·5H₂O, 8mg MnCl₂·4H₂O, 200mg ZnCl₂/L) and cultured at 28°C and 180 rpm for 7 days. Subsequently, the culture liquid was centrifuged at 9,000 rpm for 10 minutes at 4°C. The culture supernatant separated contains cellulase produced by *Trichoderma* ATCC66589 strain, itself, β-glucosidase of *Aspergillus aculeatus* and the cellulase activator of the present invention derived from expression vector p2636A49-101 or p1028A66-101 as a mixture. The solution containing cellulase derived from *Trichoderma* ATCC66589 strain, β-glucosidase of *Aspergillus aculeatus* and the cellulase activator derived from p2636A49-101 was designated as 2636A49 enzyme solution; and the solution containing cellulase derived from the *Trichoderma,* β-glucosidase of *Aspergillus aculeatus* and the cellulase activator derived from p1028A66 was designated as 1028A66 enzyme solution and used in the following Example 3. As a control enzyme solution, an enzyme solution prepared by using *Trichoderma* ATCC66589 strain not transformed was used.

### <Example 3: Saccharification of lignocellulosic biomass>

### (Purpose)

Using the cellulase activators prepared in Example 2, saccharification effect thereof on a lignocellulosic biomass is verified.

### (Method)

### (1) Preparation of substrate biomass

Rice straw was subjected to a hydrothermal treatment in accordance with a method described in JP Patent Publication (Kokai) No. 2012-139211A. After the treatment, the rice straw was broken for 5 minutes by a food mixer and passed through a 100 µm-mesh sieve to recover particles of 100 µm or less. To the particles recovered, Milli-Q water was added. The mixture was stirred while heating in a hot bath of 60°C for one hour or more. Thereafter the mixture was centrifuged at 6000 rpm and 4°C for 15 minutes and the supernatant was removed. A series of operations from recovery of particles to removal of the supernatant was repeated until a non-color supernatant was obtained. After washing, the biomass was dried by a freeze dryer, and then, the weight thereof was measured by a precision balance. A 5% (w/vol) biomass suspension was prepared with Milli-Q water.

### (2) Method for measuring biomass enzymatic saccharification

Each of the 5% biomass suspensions (100 µL) was dispensed in 96-well plates, and centrifuged at 4000 rpm and 4°C for 10 minutes (KUBOTA6930; KUBOTA) and the supernatant was removed by an aspirator. To the supernatant-free biomass, 100 mM acetic acid buffer (pH 5.0) and 2636A49 enzyme solution (1.0 mg/g-biomass) or 1028A66 enzyme solution (0.5 mg/g-biomass) prepared in Example 2 were added to prepare a reaction solution of 100 µL in volume. A sample containing 2636A49 enzyme solution was incubated for 48 hours and a sample containing 1028A66 enzyme solution for 24, 48 and 72 hours, while shaking at 1300 rpm and 45°C. Thereafter, centrifugation was carried out by using a 96-well plate filter at 4000 rpm and 4°C for 10 minutes and the supernatant was recovered.

Enzyme activity was determined by measuring the amount of reducing sugar, which was a reaction product present in the supernatant of the reaction solution, in accordance with DNS (3,5-dinitrosalicylic acid) method. The supernatant (20 µL) and DNS reagent (40 µL) were mixed. The DNS reagent used herein was prepared by adding 80 mL of 0.5 M NaOH, 0.5 g of DNS and 30 g of KNaC₄H₄O₆-4H₂O and diluting the mixture with pure water in a measuring cylinder to 100 mL. The mixture was heated at 95°C for 5 minutes and then immediately cooled in ice water. To the mixture, Milli-Q water (180 µL) was added and stirred, and absorbance thereof at 540 nm was measured by use of a plate reader (Molecular Devices: SpectraMaxM2^{e}). Based on the glucose standard solutions having a glucose concentration of 0, 0.125, 0.25, 0.5, 1.0, 1.5, 2.0 and 2.5 mg/mL, a calibration curve was prepared and the amount of free reducing sugar was obtained in terms of glucose. Note that, the DNS method was carried out using triplet samples.

### (Results)

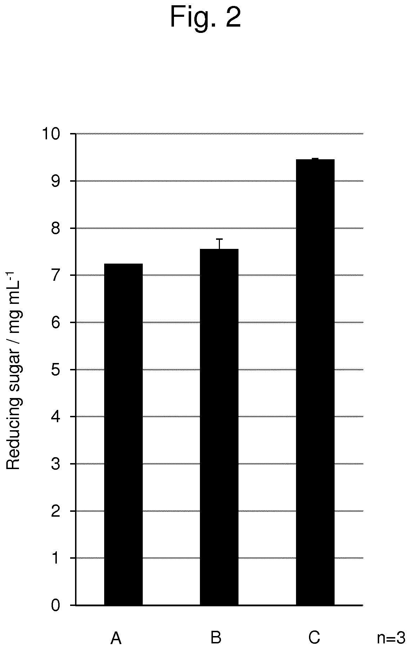

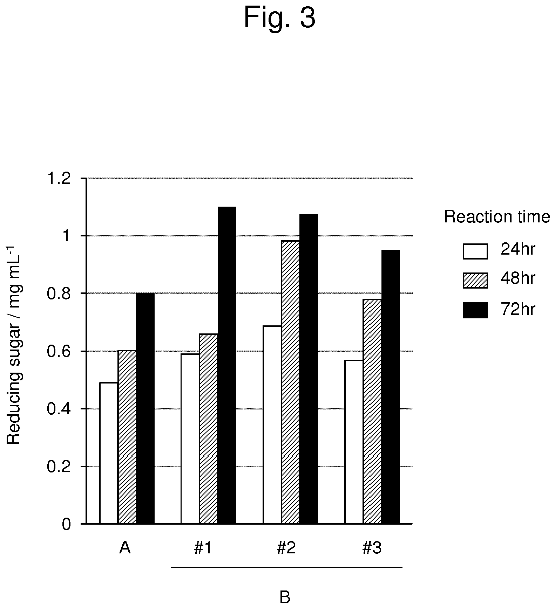

The results of the saccharification reaction of rice straw to which 2636A49 enzyme solution was added are shown in Figure 2; whereas, the results of the saccharification reaction of rice straw to which 1028A66 enzyme solution was added are shown in Figure 3.

In Figure 2, A represents commercially available Cellic (registered trade mark) CTec2 (Novozymes); B represents a control enzyme solution prepared by *Trichoderma* ATCC66589 strain transformed with pBI101; and C represents 2636A49 enzyme solution. Cellic (registered trade mark) CTec2 is known as the enzyme presently having the highest degradation activity of the commercially available lignocellulose degradation enzymes. In sample C containing 2636A49 enzyme solution, the saccharification efficiency, which was significantly higher than those in sample A and control enzyme solution B, could be obtained.

In Figure 3, A represents the results of the case where a control enzyme solution prepared by *Trichoderma* ATCC66589 strain not transformed was added; whereas B represents the results of the case where 1028A66 enzyme solution was added. B indicates the results of the cases where a reaction was carried out for 24, 48 and 72 hours after the enzyme solution was added. Reference numbers #1 to #3 represent clones independently screened as transformants of *Trichoderma* ATCC66589 strain with p1028A66. In any one of the cases, the amount of free reducing sugar was higher than the case (A) of *Trichoderma* ATCC66589 strain alone. It was also found that the amount of free reducing sugar increases in the reaction time dependent manner.

From the results, it was demonstrated that the cellulase activator of the present invention can enhance the cellulase activity and accelerate saccharification of a lignocellulosic biomass.

Note that, all publications, patents and patent applications cited in the specification are incorporated herein in their entirety by reference.

## Claims

1. A polypeptide consisting of any one of the following amino acid sequences (a) to (c) or an active fragment thereof:
(a) an amino acid sequence represented by SEQ ID NO: 1 or 9,
(b) an amino acid sequence obtained by deleting, substituting or adding one or more amino acids in the amino acid sequence represented by SEQ ID NO: 1 or 9, and
(c) an amino acid sequence having an amino acid identity of 90% or more with the amino acid sequence represented by SEQ ID NO: 1 or 9.

2. A polynucleotide encoding the polypeptide or an active fragment thereof according to Claim 1.

3. The polynucleotide according to Claim 2, consisting of any one of the following nucleotide sequences (d) to (g):
(d) a nucleotide sequence represented by SEQ ID NO: 2 or 10,
(e) a nucleotide sequence obtained by deleting, substituting or adding one or more nucleotides in the nucleotide sequence represented by SEQ ID NO: 2 or 10,
(f) a nucleotide sequence having a nucleotide identity of 90% or more with the nucleotide sequence represented by SEQ ID NO: 2 or 10, and
(g) a nucleotide sequence hybridizing with a part of the nucleotide sequence complementary with the nucleotide sequence represented by SEQ ID NO: 2 or 10, under stringent conditions.

4. An expression vector comprising the polynucleotide according to Claim 2 or 3 expressibly.

5. A transformant obtained by introducing the expression vector according to Claim 4 into a host, or a progeny thereof.

6. The transformant or a progeny thereof according to Claim 5, wherein the host is a *Trichoderma* filamentous fungus.

7. A cellulase activator comprising the polypeptide according to Claim 1 as an active ingredient.

8. A method for saccharifying a lignocellulosic biomass, comprising
a cellulase mixing step of mixing cellulase with the lignocellulosic biomass;
a cellulase activator mixing step of mixing the cellulase activator according to Claim 7 with the lignocellulosic biomass; and
a reaction step of bringing the lignocellulosic biomass and the cellulase into contact with each other in the presence of the cellulase activator to hydrolyze lignocellulose.

9. A method for producing a reducing sugar from a lignocellulosic biomass using the method according to Claim 8.


SEQUENCE LISTING

<110>  RIKEN

<120>  Cellulase enhancing agent and method for glycosylating
       lygnocellulosic biomass using the same

<130>  PH-6383-PCT

<150>  JP 2014-230027
<151>  2014-11-12

<160>  12

<170>  PatentIn version 3.5

<210>  1
<211>  227
<212>  PRT
<213>  commensal flagellate in digestive tract of Neotermes
       koshunensis and Coptotermes formosanus

<400>  1

Met Leu Ala Leu Val Ile Ala Leu Gly Val Ala Ala Pro Ile Arg Ile
1               5                   10                  15

Trp Pro Leu Gly Asp Ser Ile Thr His Arg Gly Cys Trp Arg Ala Tyr
            20                  25                  30

Leu Trp Arg Gln Leu Gln Gln Asp Gly Tyr Thr Gly Ile Asp Phe Thr
        35                  40                  45

Gly Thr Leu Asn Asp Cys Asn His Cys Asp Gln Cys Asn Asn Phe Gln
    50                  55                  60

Tyr Asp His Asp His Glu Gly His Gly Gly Phe Leu Ile Thr Asn Ile
65                  70                  75                  80

Ala Ala Gly Asn Tyr Gly Gln Asp Pro Lys Gly Gly Thr Phe Asp Gln
                85                  90                  95

Trp Ile Ala Val Gly Lys Pro Asp Ile Thr Leu Met Leu Phe Gly Thr
            100                 105                 110

Asn Asp Cys Met Ala Asn Lys Ala Thr Thr Ser Ile Ile Ser Ala Tyr
        115                 120                 125

Thr Trp Ala Leu Gln Lys Met Arg Thr Ser Asn Pro Lys Val His Leu
    130                 135                 140

Ile Val Ser Lys Val Leu Pro Val Asp Gln Ser Leu Asn Gln Ile Lys
145                 150                 155                 160

Ala Leu Asn Thr Ala Ile Asp Thr Trp Ala Ala Gln Gln Thr Thr Lys
                165                 170                 175

Asp Ser Pro Ile Val Val Val Asp Gln Phe Thr Gly Ile Asn Thr Lys
            180                 185                 190

Thr Asp Leu Tyr Asp Gly Ile His Pro Gly Asp Ser Gly Gln Gln Lys
        195                 200                 205

Tyr Tyr Lys Asn Leu Ile Lys Pro Val Glu Thr Ser Leu Asp Ala Lys
    210                 215                 220

Lys Thr Thr
225

<210>  2
<211>  684
<212>  DNA
<213>  commensal flagellate in digestive tract of Neotermes
       koshunensis and Coptotermes formosanus

<400>  2
atgttggctt tggtcatcgc ccttggcgtt gctgcgccga tccggatttg gcctcttggc      60

gactcgatca cacatcgggg ctgctggcgc gcgtatctct ggcggcagct ccagcaggac     120

ggctacacgg gcatcgattt caccgggact ctgaatgact gcaaccactg cgaccaatgc     180

aacaacttcc aatacgacca cgaccacgag ggtcatgggg gtttcctgat cacgaacatt     240

gcggcgggga actacgggca ggacccgaag gggggtacgt ttgaccagtg gatcgctgtg     300

ggaaagccgg acatcacact gatgctgttc gggacgaacg actgcatggc gaacaaggcc     360

acgacgtcga tcatcagtgc ttacacttgg gcgctgcaga agatgcggac ctcaaacccc     420

aaggtgcatc tgatcgtctc gaaggtcctg ccagttgatc agtcgctgaa ccaaatcaag     480

gcactgaaca cggcgatcga cacttgggcg gcccagcaga cgacgaagga ctctccaatc     540

gtcgtcgttg atcagttcac gggaatcaac accaagacgg acctctacga cggaatccac     600

cccggcgaca gcggccagca gaagtactac aagaacctca tcaagcccgt cgagacttcg     660

ctcgacgcta agaagacgac ctaa                                            684

<210>  3
<211>  30
<212>  DNA
<213>  Artificial

<220>
<223>  primer

<400>  3

gtgtttgaca ggatatattg gcgggtaaac                                        30

<210>  4
<211>  30
<212>  DNA
<213>  Artificial

<220>
<223>  primer

<400>  4
aatttgttta caccacaata tatcctgcca                                        30

<210>  5
<211>  31
<212>  DNA
<213>  Artificial

<220>
<223>  primer

<400>  5
tggtgtaaac aaattcagaa acctcttttg a                                      31

<210>  6
<211>  30
<212>  DNA
<213>  Artificial

<220>
<223>  primer

<400>  6
tatcctgtca aacacgcctc cattgccatt                                        30

<210>  7
<211>  895
<212>  DNA
<213>  Trichoderma sp.

<400>  7
cagaaacctc ttttgatgta gttatgcgca tgctagactg ctcctgtttc atgtggttac      60

aacaaacagt ctgatcgact tcgaatactt ggactgatga aggttgtaca gattgctgac     120

agatgtcgta atgcagagca aggctgtaga ttccataaaa ccagttgctt cgcctgctgt     180

ggctctggag aaccaaagag acgtgtctcg ggagggtaag tggtatcgaa tctatgagag     240

aagcccagtc taagagagga ccatctcgcc aggggaagat gaagctggtt acaactgatt     300

tgttttcccg tctgccacca tggtatagag cctggaccaa tcaggctaaa tcattgtata     360

caataagcct agagaaaacc tgaaatctgt cctcgtcctt tgtccgttgt ctaattatcc     420

gttattttcg aacgatgata cagtatgagt tttgccgaaa ttttgctaaa ggtactatcg     480

acgggggaca caagggttga gtctgtataa cggctcgaaa cagcagctgg tagcaggaat     540

ccaggcccgc gtttcatttg gattcatttt cccatattcc ccttgcagaa ggatacgaca 600

gtagcattgg aaaccgtaaa tgacggcaaa aagcatggtt ctgctcagat actccaagcc 660

aacctatcgg gtcctggagg ctatttccaa catctcatag cctaacagaa ataacggaag 720

tcggcatctg tatcgctcaa actgaccaga cgagcccgcc atatcgaggc agagttactc 780

tgtgttgcaa atccaactta taaagacaac aaccgcaaac tttgtcttgt cgccatcaga 840

ttgttcgcca agcaccctcc ccccccccta tcttagtcct tcttgttgtc ccaaa 895

<210> 8
<211> 858
<212> DNA
<213> Trichoderma sp.

<400> 8
agcgttgact tgcctctggt ctgtccagac gggggcacga tagaatgcgg gcacgcaggg 60

agctcgtaga cattgggctt aatatataag acatgctatg ttgtatctac attagcaaat 120

gacaaacaaa tgaaaaagaa cttatcaagc actgtaccaa ggaagctcat tatgcgtctg 180

gcgggttcaa atgatccgtg ataggttatg ccagctgatt gtttgcccgc agggttgaca 240

ccaccagggg ataatggcca ctttcatctg aatcaaggac aggagccgtt gatgatttcc 300

agatatccta tcttcatcgc tagtactaat aacaagtaag caaacagcca gctacactcg 360

tacacaccgc tcatgaaaac ataagacaaa gctcaagcca tgtcaagcac caccccatcg 420

tacacgtaca tcttctacca atctgtccag atgccggcta cgtcagcctc caacccattg 480

cgatacaatg acggatgtcg tatagaccat ggcaacggct cccaccacca ttgtcactgc 540

gtccaaagtc ttctcccacc tgctctcagc aacgcccttg aaatgcagga atgccggata 600

catgtagacc aatggaatgc aggcaaaaga tcccgtcagc gccacaaact tatccaaatc 660

acttgctccc accaccgcca cgccgatgca tgccacaatg gtcaaggccc ggaggccgtt 720

cttcttccac ttgattgccg cgctcttctt accagtggcg cgctctccga aaatcgacgt 780

ttccaatatg cggatcgcgg ggaacagctg cacaggctcg ccagccagta cggccagcga 840

gtagagaaac tggactgc 858

<210> 9
<211> 214
<212> PRT
<213> commensal flagellate in digestive tract of Neotermes
      koshunensis and Coptotermes formosanus

<400> 9

Met Leu Gly Val Leu Ala Gly Ala Val Phe Ala Ala Gln Ile Ala Ile
1               5                   10                  15

Ser Ile Asp Asp Gly Pro Ser Ala Met Ala Gly Gln Tyr Leu Ser Val

20                  25                  30

Leu Ser Gln Lys Gly Val His Ala Thr Phe Phe Trp Ile Gly Gln Asn
        35                  40                  45

Val Arg Ser Asn Gly Gly Ser Ala Ser Ser Thr Ile Ser Gly Gly His
    50                  55                  60

Glu Val Gly Cys His Ser Asp Asn Tyr Asp Ser Met Gly Ser Asn Ser
65                  70                  75                  80

Val Ser Gln Asp Gln Ser Leu Leu Ser Ala Ala Ile Ser Ala Met Gln
                85                  90                  95

Ser Ala Gly Ala Pro Ala Pro Lys Leu Phe Arg Ala Pro Asn Leu Asp
            100                 105                 110

Tyr Ser Thr Thr Leu Leu Gln Ala Ala Gly Gln Leu Gly Met Ala Val
        115                 120                 125

Ile Gly Thr Asp Val Leu Gly His Asp Trp Glu Ser Gly Val Gly Thr
    130                 135                 140

Ser Gln Ile Val Asn Tyr Ile Val Gln Ala Ala His Asp Gly Gly Ile
145                 150                 155                 160

Ile Leu Leu His Glu Gln Ser Ser Gly Asp Thr Lys Ser Val Glu Ala
                165                 170                 175

Leu Gly Gln Ile Ile Asp Gln Leu Lys Ala Lys Gly Tyr Glu Phe Phe
            180                 185                 190

Thr Val Ser Gly Leu Ala Gln Ala Lys Gly Val Asn Leu Gln Ala Gly
        195                 200                 205

Thr Arg Tyr Asp Lys Phe
    210

<210>  10
<211>  645
<212>  DNA
<213>  commensal flagellate in digestive tract of Neotermes
       koshunensis and Coptotermes formosanus

<400>  10
atgctggggg tactggcggg agcggtgttc gcggcgcaga tagcgatctc aatagacgat      60

gggccgagtg caatggcagg ccagtacctg agtgtgttgt cgcagaaggg agtgcatgcg     120

acgtttttct ggatagggca gaacgtgcgt tcgaacggag ggagtgcatc atcaacgatc     180

tcgggaggtc atgaggtggg gtgccactcg gacaactatg attcgatggg gagcaacagt 240

gtgtcgcagg atcagtcgct gctgtcggcg gcgataagtg caatgcagtc ggcgggagcg 300

ccggcgccga agttgttccg agcgccgaac ctggactaca gcacgacgct gctgcaggcg 360

gcgggtcagt tggggatggc ggtgatcggg acggatgtgt tgggtcatga ttgggagagt 420

ggagtgggga cgtcgcagat agtgaattac atagtgcagg cggcgcatga tggagggatc 480

atcctgctgc acgagcagag ttcgggcgac acgaagtcgg tggaggcgct gggtcaaata 540

atagatcagc tgaaggcgaa ggggtatgag ttcttcacgg tgagtgggct ggcgcaggcg 600

aagggagtga atttgcaggc gggtacgcgg tatgacaagt tttga 645

<210> 11
<211> 30
<212> DNA
<213> Artificial

<220>
<223> primer

<400> 11
tcagtcgtca gcgctgcgca gatagcgatc 30

<210> 12
<211> 30
<212> DNA
<213> Artificial

<220>
<223> primer

<400> 12
gtggtggtgg tggtgaaact tgtcataccg 30